# EUROPEAN PATENT APPLICATION

(11) **EP 0 747 375 A1**
(43) Date of publication of application: **11.12.1996**
(21) Application number: 96303773.4
(22) Date of filing: 28.05.1996
(51) Int. Cl.: C07D 317/34

(54) **Method for making orthoester monomers**

(30) Priority: 05.06.1995 US 461469
(71) Applicant: GENERAL ELECTRIC COMPANY, Schenectady, NY 12345 (US)
(72) Inventor: Khouri, Farid Fouad, Clifton Park, New York 12065 (US)
(74) Representative: Szary, Anne Catherine

(57) **Abstract**

A method for making substituted orthoester monomers comprising the step of contacting diols having at least one graftable pi bond such as 1-allyloxy-2,3-propanediol, and orthocarboxylates such as trimethylorthoacetate in the absence or presence of inert solvents.

## Description

### Field of the Invention

The instant invention is directed to a process for making substituted orthoester monomers. The process is a single step process which unexpectedly results in superior product yield as well as improved product purity.

### Background of the Invention

In recent years, it has been desirable to produce polymers having orthoester functional groups. The polymers bearing orthoester functional groups are often prepared by contacting the polymers with ethylenically unsaturated cyclic orthoesters. The ethylenically unsaturated cyclic orthoesters have, in the past, been produced by methods which result in moderate yields and undesirable by-products. Moreover, such production is a cumbersome two step process which typically requires the use of reagents such as solid sodium hydroxide which is often very difficult to handle.

It is of increasing interest, therefore, to prepare substituted orthoester monomers by a method which is not a cumbersome two step process and does not require the use of reagents which are extremely difficult to handle. Moreover, it is of increasing interest to prepare such monomers via a process resulting in superior product yield and improved product purity.

### Description of the Prior Art

Efforts have been disclosed for preparing orthoester monomers. In U.S. Patent 5,231,197, the disclosure of which is incorporated herein by reference, ethylenically unsaturated orthoester monomers are prepared via a process which requires reacting a hydroxy orthoester with electrophilic reagents, alkaline reagents and phase transfer catalysts producing the desired ethylenically unsaturated orthoesters.

### Summary of the Invention

The instant invention is directed to a process for making substituted orthoester monomers comprising the step of contacting:
(a) diols having at least one graftable pi bond; and
(b) orthocarboxylates.

### Detailed Description of the Preferred Embodiments

The diols which may be employed in this invention are not limited. In tact, any diol capable of reacting with the orthocarboxylates to produce a substituted orthoester monomer may be employed. Generally, the diols which may be employed in this invention are commercially available and they include those which are ethynyl, acryloyl, vinyl, allyl and unsaturated ether substituted. Often, the preferred diols have the formula 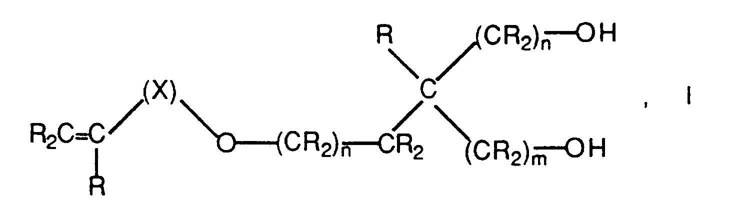 wherein each R is independently a hydrogen, halogen, C₁₋₁₀ hydrocarbon or substituted or unsubstituted aromatic radical and preferably hydrogen, m is an integer from about 0 to about 25 and preferably from about 1 to about 10 and each n is independently an integer from about 0 to about 25 and preferably from about 1 to about 10 with the proviso that m and n are not simultaneously 0 and X is a linking group and preferably a substituted or unsubstituted aromatic radical, a carbonyl group or 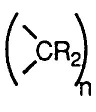 wherein R and n are as previously defined.

Moreover, it is noted herein that the diols described by formula I can be the reaction product of any unsaturated precursors capable of becoming diols after a base or acid catalyzed cleavage. Therefore, the diols include, for instance, the reaction product of unsaturated epoxy monomers which have undergone a base or acid catalyzed hydrolysis.

There is no limitation with respect to the orthocarboxylates which may be employed in this invention other than that they are capable of reacting with the diols described above to produce a substituted orthoester monomer. Such orthocarboxylates are often readily available, commercially sold, and the reaction product of, for instance, nitriles and alcohols under acidic conditions. The often preferred orthocarboxylates include, for example, trimethyl orthoacetate, triethyl orthoacetate, tripropyl orthoacetate, as well as trialkyl orthopropionates, trialkyl orthobutyrates and trialkyl orthobenzoates.

The instant invention may be carried out without a solvent (in the neat) or in the presence of a solvent. The solvents which may be employed in this invention are not limited. The only criteria is that they are inert in the presence of the diols and orthocarboxylates described above. An illustrative list of the solvents which may be employed in this invention include aliphatic hydrocarbons like pentane, hexane and the like, aromatic hydrocarbons including benzene and toluene as well as halogenated solvents such as chlorinated benzene, chloroform, dichloromethane and the like.

It is further within the scope of the instant invention to employ transorthoesterification catalysts. Such transorthoesterification catalysts are often mineral acids or organic acids and they include, for example, sulfuric acid, hydrochloric acid, nitric acid and p-toluenesulfonic acid. Additionally, acid ion exchange resins may be employed, either alone or in combination with the aforementioned mineral acids. Such acid ion exchange resins include, for example, sulfonated polystyrene ion exchange resins which are described in U.S. Patent 5,414,151, the disclosure of which is incorporated herein by reference.

When conducting the novel method described in this invention, the diols having at least one graftable pi bond, catalysts and orthocarboxylates may be added in any order to a mixing vessel. The resulting mixture may be stirred in order to produce a homogeneous mixture and enhance the reaction which is generally not limited to any temperature range. The preferred temperature is, however, about ambient temperature.

Subsequent to the single step of mixture formation, substituted orthoester monomers are formed and they may be isolated from the mixture by first adding a base such as sodium carbonate to neutralize the acid catalyst to an insoluble salt. The desired monomers may then be recovered, for instance, by removing the insoluble salt via filtration. If acid ion exchange resins are employed, they too may be removed by filtering, and neutralizing is not required. The resulting filtrate with dissolved monomer may be treated via techniques generally used to recover compounds in solution. Such techniques include evaporation and distillation. Moreover, it is noted herein that the product obtained is at least about 90% pure and preferably at least about 95% pure wherein purity is based on the amount of oligomeric by-products present. It is further noted that the reaction affords the product in at least about 90% yield and preferably in at least about 95% yield.

Additionally, when conducting the method described in this invention, it is often preferred that there is at least about a 5.0% by weight excess of orthocarboxylates present in the reaction based on the total weight of diols having at least one graftable pi bond present. With respect to the catalyst, it is often preferred that about 0.01% to about 2.0% and most preferably about 0.05% to about 0.1% by weight of catalyst is employed based on the total weight of diols having at least one graftable pi bond present. Further, the amount of base employed is at least enough to neutralize the catalyst used, when the catalyst chosen is not an acid ion exchange resin.

The substituted orthoester monomers produced via the novel method described in this invention are not limited in use and they may be employed, for example, to functionalize polymer resins. Such functionalization is described for instance in U.S. Patents 5,132,373, 5,153,290, 5,162,448 and 5,212,255, all of which are incorporated herein by reference.

The following example has been provided to further facilitate an understanding of the invention. The product obtained may be confirmed by conventional techniques including carbon-13 and proton nuclear magnetic resonance spectroscopy, mass spectroscopy and infrared spectroscopy.

### Example 1

A mixing vessel was charged with dichloromethane (1250 ml), 264.32 g (2.0 mol) of 1-allyloxy-2,3-propanediol, 0.2 g of p-toluenesulfonic acid, and 252.31 g (2.1 mol) of trimethylorthoacetate under a nitrogen atmosphere. The resulting mixture was stirred until it became homogeneous. The homogeneous mixture was allowed to stir overnight at room temperature. 2.0 g of anhydrous sodium carbonate were subsequently added and the resulting solution was stirred for 1.5 hours. A solid precipitate formed (neutralized catalyst) and was recovered by filtration. The remaining solution/filtrate having the substituted orthoester monomer was concentrated on a rotary evaporator. Vacuum distillation of the resulting monomer residue gave 360 g (96% yield) of 4-allyloxymethyl-2-methoxy-2-methyl-1,3-dioxolane (boiling point 55°C/0.3 torr).

## Claims

1. A method for making substituted orthoester monomers comprising the step of contacting:
(a) diols having at least one graftable pi bond; and
(b) orthocarboxylates.

2. A method in accordance with claim 1 wherein said contacting is in the presence of an aliphatic hydrocarbon, aromatic hydrocarbon or halogenated solvent.

3. A method in accordance with claim 1 wherein said diols having at least one graftable pi bond have the formula 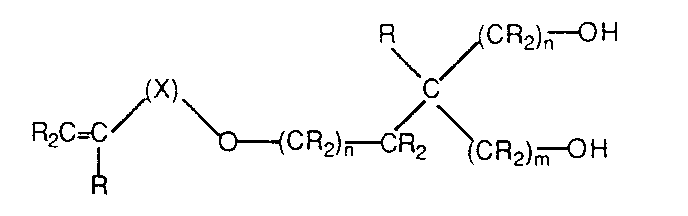 wherein each R is independently a hydrogen, halogen, C₁₋₁₀ hydrocarbon, substituted or unsubstituted aromatic radical, m is an integer from about 0 to about 25 and each n is independently an integer from about 0 to about 25, with the proviso that m and n are not simultaneously 0, and X is a linking group.

4. A method in accordance with claim 3 wherein said linking group is a substituted or unsubstituted aromatic radical, a carbonyl group or

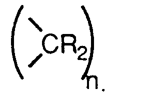

5. A method in accordance with any preceding claim wherein said orthocarboxylates are trimethylorthoacetates, triethylorthoacetates, tripropylorthoacetates, trialkylorthopropionates, trialkylorthobutyrates and trialkylorthobenzoates.

6. A method in accordance with any preceding claim wherein said method further comprises the step of adding transorthoesterificaton catalysts.

7. A method in accordance with claim 6 wherein said transesterification catalysts are mineral acids, organic acids or acid ion exchange resins.

8. A method in accordance with claim 6 wherein about 0.01% to about 2.0% by weight transorthoesterification catalyst is employed based on total weight of diols having at least one graftable pi bond present.

9. A method in accordance with any preceding claim wherein said substituted orthoester monomers are 4-alloyloxymethyl-2-methoxy-2-methyl-1,3-dioxolane monomers.

10. A method in accordance with any preceding claim wherein said orthocarboxylates are present in an amount of at least about a 5.0% by weight excess based on total weight of diol having at least one graftable pi bond.
